(19) Europäisches Patentamt
European Patent Office
Office européen des brevets

(11) Veröffentlichungsnummer: **0 013 910**
**B1**

(12) # EUROPÄISCHE PATENTSCHRIFT

(45) Veröffentlichungstag der Patentschrift: **08.12.82**

(51) Int. Cl.³: **A 61 K 39/385,** G 01 N 33/56, C 07 G 7/00

(21) Anmeldenummer: **80100116.5**

(22) Anmeldetag: **11.01.80**

(54) **Theophyllin-Antigene und -Antikörper, Verfahren zu deren Herstellung, ihre Verwendung zur Bestimmung von Theophyllin, Reagens-Set und Antiserum für diese Verwendung.**

(30) Priorität: **13.01.79 DE 2901218**

(43) Veröffentlichungstag der Anmeldung: **06.08.80 Patentblatt 80/16**

(45) Bekanntmachung des Hinweises auf die Patenterteilung: **08.12.82 Patentblatt 82/49**

(84) Benannte Vertragsstaaten: **DE FR IT**

(73) Patentinhaber: **Byk Gulden Lomberg Chemische Fabrik GmbH, Byk-Gulden-Strasse 2, D-7750 Konstanz (DE)**

(72) Erfinder: **Zech, Karl, Dr. Dipl.-Chem., Am Guckenbühl 17, D-7750 Konstanz 16 (DE)**
Erfinder: **Amschler, Hermann, Dr. Dipl.-Chem., Hohenhewenstrasse 19, D-7760 Radolfzell (DE)**

(56) Entgegenhaltungen:
**DE-A-2 805 961**
**FR-A-2 309 236**
**US-A-4 104 367**

**CHEMICAL ABSTRACTS, Vol. 84, Nr. 21, 24. Mai 1976, Zusammenfassung Nr. 144484t, Seiten 8, 9, Columbus, Ohio, US, C. E. COOK et al.: »Theophylline radioimmunoassay: synthesis of antigen and characterization of antiserum«**

**CHEMICAL ABSTRACTS, Vol. 87, Nr. 16, 17. Oktober 1977 Zusammenfassung Nr. 122685u, Seite 321, Columbus, Ohio, US, O. YU. POLEVAYA et al.: »Synthesis and characteristics of conjugated caffeine-protein antigens«**

**JOURNAL OF ORGANIC CHEMISTRY, Vol. 41, Nr. 3, 1976, B. N. HOLMES et al.: »Allylic re-arrangment from 06 to N-3 and N-7 of Guanine Blocked at C-8«, Seiten 568—570**

**Die Akte enthält technische Angaben, die nach dem Eingang der Anmeldung eingereicht wurden und die nicht in dieser Patentschrift enthalten sind.**

**EP 0 013 910 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

# Theophyllin-Antigene und -Antikörper, Verfahren zu deren Herstellung, ihre Verwendung zur Bestimmung von Theophyllin, Reagens-Set und Antiserum für diese Verwendung

Theophyllin (1,3-Dimethylxanthin) ist ein weitverbreitetes Arzneimittel, das insbesondere zur Behandlung des bronchitischen Syndroms, aber auch zur Behandlung von Bluthochdruck und des Nierenödems verwendet wird. Des weiteren wird es zur Behandlung der temporären Asphyxie unreifer Neugeborener eingesetzt. Wegen der relativ geringen Breite des therapeutischen Bereichs dieses Arzneimittels und des von Patient zu Patient stark variierenden Serumspiegels besteht ein Bedürfnis nach einer empfindlichen und wenig aufwendigen Analysenmethode zur individuellen Einstellung und Überwachung des Theophyllin-Serumspiegels. Dieses Analysenverfahren soll möglichst wenig von den normalen Metaboliten des Theophyllin und anderen im Serum vorkommenden Xanthinderivaten, wie z. B. Coffein, beeinflußt werden.

In Research Communications in Chemical Pathology and Pharmacology 13, 497 (1976) wird ein Verfahren zur Gewinnung eines Theophyllin-Antiserums beschrieben, das durch Immunisierung von Kaninchen mittels eines Konjugats aus Theophyllin-8-buttersäure und Rinderserumalbumin gewonnen wird. Aus J. Pharmacol. Exp. Ther. 199, 679 (1976) ist ein Verfahren zur Gewinnung von für Koffein spezifischen Antikörpern bekannt, das auf der Immunisierung von Kaninchen mittels an Rinderserumalbumin gekoppelter Theophyllin-7-capronsäure beruht.

Ferner ist aus der DE-A-2 805 961 bekannt, das Theophyllin in 3-Stellung zu entsprechenden Antigenen zu koppeln, die aber eine vergleichsweise geringere Spezifität aufweisen.

Es wurde nun überraschenderweise gefunden, daß sich mit Hilfe von in 7-Stellung substituiertem Theophyllin Immunoassays zur Bestimmung von Theophyllin herstellen lassen, die sich insbesondere durch ihre überlegene Empfindlichkeit auszeichnen.

Gegenstand der Erfindung sind Antigene bestehend aus einer Verbindung der allgemeinen Formel I

$$T-(CH_2)_n-COOH \qquad (I)$$

worin T einen Theophyllin-7-Rest und n eine ganze Zahl von 2 bis 4, bevorzugt von 3 bis 4 und besonders die 3 bedeutet, wobei die Verbindung über die Carboxylgruppe kovalent in einen immunogenen Träger, insbesondere ein Protein, vorzugsweise Rinderserumalbumin, gebunden ist.

Die Herstellung der Verbindungen der allgemeinen Formel I erfolgt in an sich bekannter Weise.

Zur Herstellung der Theophyllin-7-alkancarbonsäuren wird Theophyllin in ein Metallsalz, vorzugsweise Alkalisalz übergeführt. Dies geschieht zweckmäßigerweise in einem inerten, wasserfreien Lösungsmittel, wobei insbesondere Dimethylformamid, Dimethylacetamid und Acetonitril in Frage kommen, durch Umsetzung mit einem Metallhydrid, vorzugsweise Alkalihydrid, wie beispielsweise Natriumhydrid oder auch Lithiumhydrid. Das Theophyllinsalz wird mit einem funktionellen Derivat einer n-Alkancarbonsäure, vorzugsweise deren Ester, die in ω-Stellung eine nucleofuge Austrittsgruppe trägt, alkyliert. Unter einer nucleofugen Austrittsgruppe wird insbesondere ein Chlor-, Brom- oder Iodatom, vorzugsweise ein Bromatom, oder eine Sulfonyloxygruppe, wie z. B. p-Toluolsulfonyloxy-, Benzolsulfonyloxy- oder Methansulfonyloxygruppe, vorzugsweise eine Toluolsulfonyloxygruppe, verstanden. Das erhaltene Theophyllin-7-alkancarbonsäurederivat wird anschließend auf eine dem Fachmann geläufige Weise in die freie Säure oder ein Salz derselben überführt; Die Verseifung eines Theophyllin-7-alkancarbonsäureesters erfolgt z. B. durch Alkalihydroxid, insbesondere Kaliumhydroxid.

Bevorzugt wird Theophyllin-7-buttersäure hergestellt.

Die kovalente Bindung der als Haptene zu bezeichnenden Theophyllin-7-alkancarbonsäuren der allgemeinen Formel I an einem immunogenen Träger erfolgt auf an sich bekannte Weise. Als immunogener Träger können verschiedene Arten von Proteinen und Polypeptiden verwendet werden, die bei einer Injizierung in Wirtstiere selbsttätig eine Immunreaktion im Wirtstier hervorrufen. Bevorzugte Beispiele sind Säugetier-Serumproteine, wie menschliches γ-Globulin, menschliches Serumalbumin, Hasenserumalbumin, Rinder-γ-Globulin und Rinderserumalbumin. Letzteres gehört im Rahmen dieser Erfindung zu den besonders bevorzugten immunogenen Trägern. Obwohl nicht zwingend, werden vorzugsweise solche Proteine verwendet, die für das Wirtstier artfremd sind. Außer Proteinen können synthetische Polypeptide, wie z. B. Polymere von Lysin, Glutaminsäure und dergleichen, entweder einzeln oder in Kombination verwendet werden.

Die kovalente Bindung einer Verbindung der Formel I an einem immunogenen Träger kann in der für die Herstellung von Ester- oder Amidverbindungen üblichen Art erfolgen. In einer Ausführungsform kann z. B. eine Verbindung der Formel I vor der Kupplung an einen immunogenen Träger in eine isolierbare aktivierte Form übergeführt werden. Eine geeignete isolierbare aktivierte Form ist z. B. der N-Hydroxysuccinimidester. Ebenfalls geeignet ist der p-Nitrophenylester. In einer bevorzugten Ausführungsform erfolgt die Herstellung der aktivierten Form durch Umsetzung mit einem Carbodiimid oder insbesondere N,N'-Carbonyldiimidazol, in einem geeigneten Lösungsmittel, wie beispielsweise Dimethylformamid, Acetonitril, Tetrahydrofuran, Dimethylsulfoxid und Hexamethylphosphoramid. Eine besonders vorteilhafte Methode der Herstellung einer kovalenten Kupplung einer

Verbindung der Formel I an einen immunogenen Träger ist das Mischanhydrid-Verfahren. In diesem Verfahren wird eine Verbindung der Formel I in einem inerten, wasserlöslichen organischen Lösungsmittel, z. B. einem cyclischen Ether, wie Dioxan, gelöst und die Lösung mit einem Tri(niederalkyl)amin, wie z. B. Tri-(n-butyl)-amin, neutralisiert. Anschließend wird ein Halogenameisensäureniedrigalkylester, wie z. B. Chlorameisensäureisobutylester, zugegeben. Die erhaltene Lösung wird dann bei einer Temperatur zwischen 0 bis 8°C einer Lösung des immunogenen Trägers in Wasser oder in einer z. B. 1 : 1-Mischung von Wasser und einem wasserlöslichen organischen Lösungsmittel (z. B. ein cyclischer Ether, wie Dioxan) zugegeben, wobei die Lösung des immunogenen Trägers vorher mit Hilfe einer Lösung eines Alkalimetallhydroxids, wie z. B. Natriumhydroxid, alkalisch gemacht wird. Die Kupplungsreaktion wird während einer Zeitdauer, welche zwischen einer halben Stunde und einem halben Tag liegt, fortgesetzt. Nach Dialyse, Ansäuerung und Zentrifugieren wird das gewünschte Antigen erhalten.

Gegenstand der Erfindung ist auch ein Verfahren zur Herstellung eines Antigens, dadurch gekennzeichnet, daß man eine Verbindung der allgemeinen Formel I

$$T-(CH_2)_n-COOH \qquad (I)$$

worin T einen Theophyllin-7-Rest und n eine ganze Zahl von 2 bis 4 bedeutet, mit einer Hydroxylgruppe eines immunogenen Trägers verestert oder mit einer Aminogruppe eines immunogenen Trägers in ein Säureamid überführt.

Die mit Hilfe des obigen Verfahrens hergestellten Antigene können verwendet werden, um die Bildung von gegenüber Theophyllin spezifischen Antikörpern in Wirtstieren zu induzieren, indem man das Antigen, vorzugsweise unter Zusatz eines üblichen Hilfsstoffes, wie insbesondere dem »kompletten Freundschen Adjuvans«, einschließlich eines Puffers, vorzugsweise Phosphatpuffer vom pH 7,4, diesen Wirtstieren injiziert. Geeignete Wirtstiere sind z. B. Säugetiere, wie Kaninchen, Pferde, Ziegen, Meerschweinchen, Ratten, Rinder oder Schafe. Kaninchen werden in der vorliegenden Erfindung bevorzugt. Durch wiederholtes Injizieren kann die Antikörperbildung verstärkt werden, wodurch Antisera mit hohem Antikörpertiter erhalten werden. Die die Antikörper enthaltenden Antisera werden auf übliche Weise aus dem Blut der Wirtstiere gewonnen.

Die so gewonnenen Antikörper zeichnen sich durch ihre überlegene Empfindlichkeit aus. Überraschenderweise gestattet sie eine einwandfreie Theophyllinbestimmung auch direkt aus Vollblut und haemolytischem Blut. Als ebenfalls besonders vorteilhaft ist die Tatsache zu werten, daß das Vollblut nach der Entnahme soweit verdünnt werden kann, daß kein Abbau des Theophyllins zu befürchten ist; in diesen verdünnten Proben ist auch nach mehrtätiger Lagerung bei Zimmertemperatur mit dem erfindungsgemäßen Verfahren eine genaue Bestimmung des Theophyllinspiegels möglich. Die Blutproben können somit nach der Entnahme verdünnt werden und ohne besondere Vorkehrungen bezüglich der Temperatur, wie z. B. Kühlung, an den Ort, an dem die erfindungsgemäße Theophyllinbestimmung durchgeführt wird, geschickt werden.

Ein weiterer Gegenstand der vorliegenden Erfindung ist daher die Verwendung eines Antikörpers, erhalten nach dem vorstehend beschriebenen Verfahren zur Bestimmung von Theophyllin.

Dieses Verfahren zur Bestimmung von Theophyllin in einer Probe ist ebenfalls Gegenstand dieser Erfindung und ist dadurch gekennzeichnet, daß man die Probe mit einer bekannten Menge von markiertem Theophyllin und einem Antikörper, der mit Theophyllin unter Komplexbildung reagiert und welcher durch Injektion eines der weiter oben beschriebenen Antigene in Wirtstiere erzeugt wurde, vermischt, den Bindungsgrad des markierten Theophyllin mißt und aus dieser Messung die in der Probe vorhandene Menge Theophyllin durch Vergleich mit einer durch Hinzufügen bekannter Mengen von Theophyllin zu einem definierten Gemisch aus markiertem Theophyllin und dem Antikörper erhaltenen Standardkurve bestimmt. Diese als Immunoassay bezeichnete Methode ist dem Fachmann an sich geläufig.

Besondere Ausgestaltungen dieses erfindungsgemäßen Verfahrens sind solche, in denen radioaktiv markiertes, enzym- oder fluoreszenzmarkiertes Theophyllin bzw. Theophyllinderivate verwendet werden.

Eine weitere Ausgestaltung der Erfindung ist ein Reagens-Set zur Verwendung in dem vorstehend beschriebenen Verfahren, das dadurch gekennzeichnet ist, daß es markiertes Theophyllin und Antikörper, gewonnen nach dem weiter vorn beschriebenen Verfahren, enthält. In einer bevorzugten Ausführungsform enthält dieses Reagens-Set außerdem, wie es in derartigen Reagensbestecken des Handels üblich ist, sämtliche für die Durchführung einer Bestimmung notwendigen Hilfsreagenzien, wie z. B. Standardlösungen, Pufferlösungen, Präzipitierungsmittel und Agglutinierungsmittel in gebrauchsfertiger Form.

Das erfindungsgemäße Immunoassay zeichnet sich neben der bereits erwähnten überlegenen Empfindlichkeit vor allem durch seine große Spezifität aus. Es zeigt geringere Kreuzreaktionen mit den potentiell in den zu untersuchenden Flüssigkeiten neben Theophyllin vorhandenen Purinkörpern aus. So beträgt beispielsweise die Kreuzreaktion mit Coffein nur 2,5%. Außerdem zeigen die Reagentien des erfindungsgemäßen Immunoassays eine sehr gute Haltbarkeit, so daß die bei einer Meßreihe nicht aufgebrauchten Anteile für spätere Theophyllinbestimmungen weiterverwendet werden können.

Unter markiertem Theophyllin sind Theophyllin und Theophyllinderivate zu verstehen, die mit den auf dem Gebiet der Immunoassay-Verfahren üblichen Markierungsmitteln markiert sind. Neben der radioaktiven Markierung (Radioimmunoassay) kommen insbesondere die Markierung durch Enzyme (Enzymimmunoassay) und durch fluoreszierende Stoffe (Fluoroimmunoassay) in Frage.

Unter radioaktiv markiertem Theophyllin sind Theophyllin und Theophyllinderivate zu verstehen, die mit Radioisotopen, wie z. B. Tritium ($^3H$), Kohlenstoff 14 ($^{14}C$), Iod 125 ($^{125}J$) oder Iod 131 ($^{131}J$) markiert sind. Als $^3H$-markiertes Theophyllin ist 8-$^3H$-Theophyllin bevorzugt, das auf bekannter Weise durch Reduktion von 8-Bromtheophyllin mittels Tritium ($^3H_2$) an Palladium/Kohle hergestellt wird. Als mit Iod$^{125}$ oder Iod$^{131}$ markiertes Theophyllin kommen im Rahmen dieser Erfindung insbesondere aktive Iodierungsprodukte von Theophyllin-7-alkancarbonsäure-N-(2-imidazol-4-yl-ethyl)-amiden, -N-[2-(4-hydroxyphenyl)-ethyl]-amiden und -N-[1-carboxy- bzw. -methoxy- oder -ethoxycarbonyl-2-(4-hydroxyphenyl)-ethyl]-amiden in Frage. Ferner kommen die entsprechenden Theophyllin-8-buttersäureamide in Frage. Bevorzugt ist mit $^{125}$Iod markiertes Theophyllin-7-buttersäure-N-(2-imidazol-4-yl-ethyl)-amid und insbesondere mit $^{125}$Iod markiertes Theophyllin-7-buttersäure-N-[2-(4-hydroxyphenyl)-ethyl]-amid.

Die Herstellung dieser Theophyllin-7-alkancarbonsäureamide aus Verbindungen der allgemeinen Formel I durch Umsetzung mit Tyramin, Histamin und Tyrosin bzw. dessen Methyl- oder Ethylester erfolgt nach dem Fachmann für die Herstellung von Carbonsäuren geläufigen Verfahren, wie sie z. B. in Houbon-Weyl, Methoden der organischen Chemie, Band XI/2, Seite 3–25 (1958) und Band XV, Seite 1–396 (1974) beschrieben sind.

Unter enzymmarkiertem Theophyllin sind Theophyllin und Theophyllinderivate zu verstehen, die an ein Enzym gekoppelt sind. Als Enzyme kommen beispielsweise in Frage Katalase, Peroxidase, $\beta$-Glukuronidase, $\beta$-D-Glukosidase, $\beta$-D-Galaktosidase, Urease, Malatdehydrogenase, alkalische Phosphatase, Glukose-oxidase und Galactose-oxidase. Besonders geeignet sind Enzyme, die Umwandlungen von Substraten katalysieren, bei denen gefärbte Produkte entstehen oder verschwinden. Die Bestimmung des Enzyms ist dann auf einfache Weise durch eine colorimetrische Bestimmung möglich. Bevorzugt ist die Kopplung von Theophyllinderivaten an Glukose-6-phosphatdehydrogenase. In diesem Fall erfolgt die Bestimmung der Menge des Konjugats aus Theophyllinderivat und Enzym durch Umsetzung mit Nikotinamidadenin-dinucleotid (NAD). Das entstehende NADH kann anhand der Absorption bei 340 nm spektralphotometrisch bestimmt werden. Als Theophyllinderivate, die an Glukose-6-phosphat-dehydrogenase gekoppelt werden können, sind Theophyllin-7- oder -8-buttersäure-N-(2-aminoethyl)-amid und 7- oder 8-(4-Amino-n-butyl)-theophyllin bevorzugt. Die Kopplung von Theophyllinderivaten an Enzyme erfolgt auf an sich bekannte Weise. Vorzugsweise werden Theophyllinderivate mit mindestens einer freien Aminogruppe gewählt, die über eine freie Carboxylgruppe des Enzyms an dieses gekoppelt werden. Diese Kopplung zwischen Aminogruppen und Carboxylgruppen wird durch Reaktionen, wie sie aus der Peptidchemie bekannt sind, durchgeführt. Spezielle Beispiele solcher Kopplungen von Haptenen mit Proteinen sind z. B. in Methods in Immunology and Immunochemistry, Band 1, beschrieben. Die Bestimmung von Stoffen auf enzymimmunologischem Weg ist dem Fachmann bekannt, beispielsweise aus Sharpe et al., Clin. Chem. 22, 733–738 (1976), den deutschen Patenten 2 155 658 und 2 164 768 sowie den deutschen Offenlegungsschriften 2 545 980 und 2 834 141.

Unter fluoreszentmarkiertem Theophyllin sind Theophyllin und Theophyllinderivate zu verstehen, die an eine fluoreszierende Substanz gebunden sind. Als fluoreszierende Stoffe kommen beispielsweise in Frage der Dansylrest (5-Dimethylamino-1-naphthalinsulfonylrest) und andere substituierte Naphthaline, o-Phthaldialdehyd, Pyrrolin-4-on, der Dimethoxyanthracensulfonylrest, 7-Methoxy-cumarin, 4-Methyl-7-methoxy-cumarin und andere Cumarinderivate, sowie Fluorescein. Als Theophyllinderivate, die fluoreszenzmarkiert werden können, sind Theophyllin-7- oder -8-buttersäure, deren 2-Aminoethylamid, sowie 7- oder 8-(4-Amino-n-butyl)-theophyllin bevorzugt. Als Fluoreszenzmarker sind 4-Methyl-7-methoxy-cumarin und Fluorescein bevorzugt. Die Kopplung des Theophyllinderivats an den Fluoreszenzmarker erfolgt in an sich bekannter Weise. In Chard et al., Clin. Chem. 25, 973–975 (1979) wird beispielsweise die Markierung einer Substanz durch Fluorescein über Fluoresceinisothiocyanat beschrieben. Die Bestimmung des fluoreszenzmarkierten Theophyllin erfolgt auf übliche Weise in einem Spektrofluorometer. Die Herstellung von Theophyllin-7- oder -8-buttersäure-N-(2-aminoethyl)-amid erfolgt durch Umsetzung von Theophyllin-7- bzw. -8-buttersäuremethylester in einem Überschuß von Ethylendiamin.

Die neuen Antigene und Antikörper der vorliegenden Erfindung können in Verbindung mit üblichen Zusatzstoffen, Puffern, Stabilisatoren, Verdünnungsmitteln verwendet werden.

Die Erfindung wird anhand der nachstehenden Beispiele veranschaulicht.

## Beispiele

### Beispiel 1

Herstellung von Theophyllin-7-buttersäure

a) Theophyllin-7-buttersäureethylester
Zu 18,0 g (0,1 Mol) Theophyllin, gelöst in 150 ml wasserfreiem Dimethylformamid, werden bei Raumtemperatur 4,8 g (0,1 Mol) 50%iges Natriumhydrid unter Rühren zugegeben. Nach beendeter Wasserstoffentwicklung tropft man unter ständigem Rühren 21,5 g (0,11 Mol) 4-Brombuttersäureethylester zu und erhitzt anschließend 2 Stunden auf 100° C. Die Lösung wird im Vakuum eingedampft und der Rückstand in Methylenchlorid aufgenommen. Die erhaltene Lösung wird vom ausgeschiedenen Natriumbromid abfiltriert, zweimal mit Wasser ausgeschüttelt, über wasserfreiem Natriumsulfat getrocknet und im Vakuum eingedampft. Die erhaltene Kristallmasse wird aus Isopropanol umkristallisiert. Man erhält 25,2 g Ester (86,4% der theoretisch berechneten Menge) vom Schmp. 79 – 80° C.

b) Verseifung des Esters
21,1 g (0,07 Mol) Theophyllin-7-buttersäureethylester werden in 100 ml Ethanol mit 6,17 g (0,11 Mol) Kaliumhydroxid 15 Minuten am Rückfluß gekocht. Die Lösung wird im Vakuum eingedampft, der Rückstand in Wasser gelöst und mit 2n Salzsäure so lange angesäuert, bis sich der Niederschlag nicht mehr vermehrt. Die ausgeschiedene Säure wird abgesaugt und aus Ethanol umkristallisiert. Man erhält 13,8 g (72,3% der theoretisch berechneten Menge) Theophyllin-7-buttersäure vom Schmp. 168 – 169° C.

### Beispiel 2

Herstellung von Theophyllin-7-buttersäure-N-[2-(4-hydroxyphenyl)-ethyl]-amid

500 mg Theophyllin-7-buttersäure werden zusammen mit 326 mg Tyraminhydrochlorid, 259 mg 1-Hydroxybenztriazol und 0,26 ml Triethylamin in 40 ml wasserfreiem Dimethylformamid gelöst. Unter Rühren werden dazu 391 mg Dicyclohexylcarbodiimid, gelöst in 10 ml wasserfreiem Dimethylformamid, zugetropft. Die Mischung wird bei 25° C 22 Stunden gerührt, vom abgeschiedenen Harnstoff abfiltriert und im Vakuum eingedampft. Der Rückstand wird in Methanol, dem 10% Chloroform zugesetzt sind, aufgenommen und auf vier PSC-Fertigplatten Kieselgel 60 $F_{254}$ (Fa. Merck-Darmstadt) aufgetragen. Nach der Entwicklung mit Chloroform/Methanol 9 : 1 werden die Substanzzonen mit dem Rf = 0,39 ausgekratzt und das Amid aus dem Kieselgel mit Methanol eluiert. Die Methanollösung wird im Vakuum eingedampft, wobei ein farbloses zähes Öl zurückbleibt, das bei Anreiben mit Ether kristallisiert. Man erhält auf diese Weise 100 mg Theophyllin-7-buttersäure-N-[2-(4-hydroxyphenyl)-ethyl]-amid vom Schmp. 158 – 162° C. Das Amid wird durch den Molekülpeak $\frac{m}{e} = 385$ im Massenspektrum charakterisiert.

### Beispiel 3

$^{125}$Iod-markiertes Theophyllin

2 µg Theophyllin-7-buttersäure-N-[2-(4-hydroxyphenyl)-ethyl]-amid, 2 µg Theophyllin-7-buttersäure-N-(2-imidazol-4-yl-ethyl)-amid oder 2 µg Theophyllin-7-butyryl-tyrosin bzw. -tyrosinmethyl- oder -ethylester, gelöst in 20 µl 50 mmol Kaliumphosphatpuffer pH 7,7 bis 8,5 werden mit 0,25 – 0,5 mCi Na$^{125}$Iodid (Amersham Buchler, Braunschweig) und mit 10 µl Chloramin-T-Lösung (20 µg in 50 mmol Kaliumphosphatpuffer pH 7,7 bis 8,5) versetzt. Nach 30 – 120 Sekunden wird die Reaktion durch Zufügen von 10 µl Natriumdisulfitlösung (50 µg in 50 mmol Kaliumphosphatpuffer pH 7,7 bis 8,5) sowie von 200 µl Kaliumiodidlösung (2 mg in identischem Phosphatpuffer) gestoppt. Anschließend wird überschüssiges Iodid vom Tracer durch Chromatographie über eine Sephadex-G-10-Säule (5 × 160 mm) abgetrennt. Die Elution erfolgt dabei mit einem Phosphatpuffer, ähnlich dem zur Umsetzung verwendeten, nur daß hier noch 0,5% Rinderserumalbumin zugefügt wurden. Es werden Fraktionen von 0,5 ml gesammelt; ein Aliquot jeder Fraktion wird zur Aktivitätsbestimmung ausgezählt. Die den Iodtracer enthaltenden Fraktionen werden vereinigt und Aliquote daraus nach Verdünnung direkt im Assay eingesetzt.

### Beispiel 4

7-(4-Aminobutyl-1)-theophyllin

Zu Theophyllin, gelöst in wasserfreiem Dimethylformamid, wird bei Raumtemperatur 50%iges Natriumhydrid unter Rühren zugegeben. Nach beendeter Wasserstoffentwicklung tropft man unter ständigem Rühren N-(4-Brombutyl-1)-phthalimid, gelöst in Dimethylformamid, zu und erhitzt anschließend 2 Stunden auf 100° C. Die Lösung wird im Vakuum eingedampft und der Rückstand in Methylenchlorid aufgenommen. Die erhaltene Lösung wird vom ausgeschiedenen Natriumbromid abfiltriert, zweimal mit Wasser ausgeschüttelt, über Natriumsulfat getrocknet und eingedampft. Die erhaltene Kristallmasse kann zur Reinigung aus Ethanol umkristallisiert werden. Sie wird nun in einem Gemisch von Hydrazinhydrat/Ethanol (3 : 1) 2 Stunden zum Sieden erhitzt. Nach dem Abkühlen fügt man soviel Salzsäure zu, bis alles Phthalhydrazid ausgefallen ist. Es wird abgesaugt, die salzsaure Lösung wird alkalisch gestellt (pH 9–10) und die Titelverbindung mit Methylenchlorid extrahiert. Der Extrakt wird über Kaliumcarbonat getrocknet und im Vakuum eingedampft. Der Rückstand wird aus Essigester/Petrolether umkristallisiert.

Beispiel 5

8-(4-Aminobutyl-1)-theophyllin

a) 1,3-Dimethyl-4-(4-phthalimidobutyl-1)-amino-uracil
1,3-Dimethyl-4-chloruracil (1 Mol) und N-(4-Aminobutyl-1)-phthalamid (2,2 Mol) werden in wenig Ethanol 3 Stunden auf 80 bis 100° C erhitzt. Nach dem Abkühlen verdünnt man das Reaktionsgemisch mit 2N Salzsäure, wobei die Titelverbindung auskristallisiert. Sie wird abgesaugt, mit Wasser gewaschen und im Vakuum getrocknet.

b) 1,3-Dimethyl-4-(4-phthalimidobutyl-1)-amino-5-nitroso-uracil
Zu einer Suspension von der unter a) beschriebenen Verbindung in einer 1 : 1-Mischung aus Aceton/Chloroform wird bei 0° C unter Rühren langsam Isoamylnitrit (10% Überschuß) getropft. Anschließend wird die gleiche molare Menge 1N Salzsäure in Ethanol zugetropft und die Mischung 4 Stunden bei 0 bis 5° C gerührt, wobei sich ein feinkristalliner Niederschlag bildet. Zur Vervollständigung der Fällung wird das Gemisch mit Ether verrührt, abgesaugt und das Kristallisat mit Ether/Ethanol nachgewaschen.

c) 8-(4-Phthalimidobutyl-1)-theophyllin
Das Kristallisat aus der Stufe b) wird in n-Butanol langsam erhitzt. Bei 110 bis 120° C wird die Lösung langsam klar und entfärbt sich. Nach weiteren 10 Minuten Rückflußkochen wird die Lösung im Eisbad gekühlt, wobei die Titelverbindung bereits auskristallisiert.

d) Die unter c) beschriebene Verbindung wird in einer 3 : 1-Mischung von Hydrazinhydrat/Ethanol, wie in Beispiel 4 beschrieben, der Hydrazinolyse unterworfen und entsprechend aufgearbeitet, wobei 8-(4-Aminobutyl-1)-theophyllin erhalten wird.

Beispiel 6

Fluoreszenzmarkiertes Theophyllin

0,88 g Theophyllin-8-buttersäuremethylester werden mit 1,3 g 4-Hydroxymethyl-7-methoxy-cumarin und einigen mg 4-Toluolsulfonsäure 2 Stunden auf 190° C erhitzt. Die Schmelze wird abgekühlt, mit Acetonitril aufgekocht und das dabei entstehende Kristallpulver abgesaugt und getrocknet. Man erhält auf diese Weise 0,8 g (56% d. Th.) (4-Methyl-7-methoxy-cumarin)-(theophyllin-8)-butyrat vom Schmelzpunkt 247° C (Zersetzung).

Die Ausgangsstoffe können wie folgt hergestellt werden:

a) Theophyllin-8-buttersäuremethylester
10 g Theophyllin-8-buttersäure werden in 50 ml Methanol aufgeschlämmt und in die Suspension 1 Stunde lang HCl-Gas eingeleitet. Unter weiterem Einleiten von HCl-Gas wird die Suspension 1 Stunde am Rückfluß gekocht. Die Gas-Einleitung wird unterbrochen und die Suspension mit 13,5 ml 2,2-Dimethoxypropan versetzt. Man leitet noch weitere 20 Minuten HCl-Gas ein und läßt dann die Mischung über Nacht bei Raumtemperatur stehen. Die Suspension wird im Vakuum bis zur Trockne eingedampft. Der Rückstand wird in 300 ml Methylenchlorid aufgenommen, 2mal mit je 100 ml gesättigter Soda-Lösung und 2 mal mit je 100 ml Wasser ausgeschüttelt. Man trocknet die Methylenchlorid-Phase über geglühtem Natriumsulfat, dampft sie im Vakuum zur Trockne ein und kristallisiert den Rückstand aus Methanol um. Man erhält auf diese Weise 5,15 g (48,4% d. Th.) der Titelverbindung vom Schmelzpunkt 200–204° C.

b) 4-Hydroxy-7-methyl-cumarin
10 g 4-Brommethyl-7-methoxy-cumarin werden in 40 ml Eisessig mit 6 g Kaliumacetat (wasserfrei) 2 1/2 Stunden unter Rückfluß gekocht. Man dampft die Lösung im Vakuum zur Trockne ein, übergießt den Rückstand mit 50 ml Ethanol und 50 ml konz. Salzsäure und kocht diese Mischung 1 1/2 Stunden am Rückfluß. Man dampft erneut alle flüchtigen Anteile im Vakuum ab, nimmt den Rückstand in Chloroform auf, schüttelt diese Lösung mit verdünnter Ammoniaklösung und mit Wasser säurefrei und trocknet sie über geglühtem Kaliumcarbonat. Nach dem Verdampfen des Chloroforms im Vakuum wird der Rückstand aus Essigsäureethylester umkristallisiert. Man erhält auf diese Weise 1,9 g (24,8% d. Th.) der Titelverbindung vom Schmelzpunkt 179–182° C.

Literatur:

4-Brommethyl-7-methoxy-cumarin:
J. A. Secrist, III, J. R. Barrio und N. J. Leonard,
Biochem. Biophys. Res. Commun. 45, 1262 (1971)

Die Veresterung von Fettsäuren mit dem obigen Cumarin ist allgemein beschrieben in:

a) W. Dünges, Analytical Chemistry, 49, 442 (1977)
b) S. Lam, E. Grushka, J. Chromatography 158, 207 (1978).

Die Herstellung von (4-Methyl-7-methoxy-cumarin)-(theophyllin-7-butyrat) erfolgt auf analoge Weise.

Beispiel 7

Herstellung des Immunogens

In 200 µl Dimethylformamid werden 1 mg Theophyllin-7-buttersäure und 250 µg N,N'-Carbonyldiimidazol zugefügt. Unter leichter Stickstoffbegasung werden nach 5minütigem Schütteln 0,5 mg Rinderserumalbumin in 0,6 ml Wasser zugefügt. Man stellt den Stickstoffstrom ab und läßt das verschlossene Gefäß 5 bis 12 Stunden bei Raumtemperatur leicht schütteln. Nach dieser Zeit wird die erhaltene Lösung unter Eisbadkühlung im Wasserstrahlvakuum gegen bidestilliertes Wasser dialysiert und gleichzeitig eingeengt. Zur Dialyse werden Sartorius Kollodiumhülsen, Typ SM 13200, verwendet. Anschließend wird das Antigen so verdünnt, daß etwa eine Konzentration von 1 mg/ml erhalten wird.

Beispiel 8

Gewinnung von Antikörper

100 µl, entsprechen etwa 0,1 mg Antigen, der lt. Beispiel 4 erhaltenen Lösung werden mit 300 µl vollständigem Freundschen Adjuvans emulgiert und während mehrerer Wochen bis Monate an Kaninchen (Weiße Neuseeländer) subkutan injiziert. Die Injektion erfolgt dabei an mehreren Stellen jeweils mit ca. 50—100 µl. Den Tieren werden jeweils eine Woche nach der Injektion aus der Ohrenvene Blut entnommen und die durch Zentrifugieren und Verdünnen mit 0,1 m Kaliumphosphatpuffer (pH 8,5), unter Zusatz von 0,1% Gelatine und 0,04% Natriumazid, im Verhältnis 1 : 500 erhaltenen Antiseren werden in einem Radioimmunverfahren zur Bestimmung von Theophyllin eingesetzt.

Beispiel 9

Radioimmunverfahren zur Bestimmung von Theophyllin

Es werden folgende Reagenzien und Materialien verwendet:

1. Teströhrchen bestehend aus Wegwerf-Kulturröhrchen (12 × 75 mm)
2. Eine Lösung von $^{125}$Iod-markiertem Theophyllin nach Beispiel 3 in 100 mmolarem Kaliumphosphatpuffer pH 8,5 unter Zusatz von 0,1% Gelatine und 0,04% Natriumazid, welche ca. 15 000 Impulse/Minute/0,1 ml ergibt.
3. Antiserum verdünnt durch den unter 2 beschriebenen Puffer 1 : 2000 im Ansatz.
4. Standardlösungen von Theophyllin in Phosphatpuffer: 5, 25, 50, 100, 250 mg/ml.
5. Eine Aufschlämmung von mit Dextran beschichteter Aktivkohle (Dextran T 70 und Norit-Aktivkohle der Firma Pharmazie und Serva Feinbiochemica) in Phosphatpuffer ohne Gelatinezusatz.
6. 1 : 50 bis 1 : 200 mit Phosphatpuffer (siehe 2) verdünntes menschliches Kontrollplasma wird mit 1; 2,5; 5 und 10 µg/ml Theophyllin versetzt und daraus die Wiederfindungsrate für Theophyllin bestimmt.
7. Zu 20 µl Standardlösung bzw. Probenverdünnung werden 200 µl Antikörperverdünnung und 100 µl Theophyllin-Tracer nach Beispiel 2 zugegeben. Nach kurzem Schütteln läßt man bei 4°C 1 Stunde lang inkubieren, anschließend wird zur Abtrennung von freiem und gebundenem Theophyllin 1 ml Dextran-Kohle-Suspension zugegeben und sofort gut geschüttelt. Nach 10 Minuten wird bei 4°C zentrifugiert (15 Minuten bei ca. 2000 g) und aus dem Überstand 1 ml abgenommen und in ein Zählgläschen überführt und die Radioaktivität auf die übliche Weise bestimmt.
   Auf diese Weise können 0,1 bis 1 ng Theophyllin und auch weniger in einem Röhrchen bestimmt werden. Bei Verwendung einer Probe von 20 µl (als Serum- oder Standardverdünnung) liegt die Empfindlichkeitsgrenze bei 35 ng/ml (95% Vertrauensgrenze).
8. Werden in einem log-logit-Auswertpapier in der Abszisse die eingesetzten Konzentrationen und in der Ordinate der Faktor

$$\frac{B}{Bo} \times 100 \left( \frac{\text{Bindung Probe}}{\text{Bindung Antikörper}} \times 100 \right)$$

   eingesetzt, läßt sich damit die Standardkurve linearisieren und die Konzentration unbekannter Proben kann in üblicher Weise direkt ausgewertet werden.

Die enzymimmunologische und fluoreszenzimmunologische Bestimmung von Theophyllin erfolgt auf anlogem Weg, indem statt radioaktiv markiertem Theophyllin enzym- bzw. fluoreszenzmarkiertes Theophylline verwendet wird und statt Radioaktivitätsmessungen Enzym- bzw. Fluoreszenzmessungen durchgeführt werden.

**Patentansprüche**

1. Antigen, bestehend aus einer Verbindung der allgemeinen Formel I,

$$T-(CH_2)_n-COOH \qquad (I)$$

worin T einen Theophyllin-7-Rest und n eine ganze Zahl von 2 bis 4 bedeutet, wobei die Verbindung über die Carboxylgruppe kovalent

an einen immunogenen Träger verbunden ist.

2. Antigen nach Anspruch 1, dadurch gekennzeichnet, daß der immunogene Träger ein Protein ist.

3. Antigen nach Anspruch 2, dadurch gekennzeichnet, daß der immunogene Träger Rinderserumalbumin ist.

4. Antigen nach einem der Ansprüche 1 bis 3, dadurch gekennzeichnet, daß in der allgemeinen Formel I n die Zahl 3 bedeutet.

5. Verfahren zur Herstellung eines Antigens nach Anspruch 1, dadurch gekennzeichnet, daß man eine Verbindung der allgemeinen Formel I

$$T-(CH_2)_n-COOH \quad (I)$$

worin T einen Theophyllin-7-Rest und n eine ganze Zahl von 2 bis 4 bedeutet, mit einer Hydroxylgruppe eines immunogenen Trägers verestert oder mit einer Aminogruppe eines immunogenen Trägers in ein Säureamid überführt.

6. Verfahren zur Bestimmung von Theophyllin in einer Probe, dadurch gekennzeichnet, daß man die Probe mit einer bekannten Menge von markiertem Theophyllin und einem Antikörper, der mit Theophyllin und dem markierten Theophyllin unter Komplexbildung reagiert und welcher durch Injektion eines Antigens nach einem der Ansprüche 1 bis 4 in Wirtstiere erzeugt wurde, vermischt, den Bindungsgrad des markierten Theophyllins mißt und aus dieser Messung die in der Probe vorhandene Menge Theophyllin durch Vergleich mit einer durch Hinzufügen bekannter Mengen von Theophyllin zu einem definierten Gemisch aus markiertem Theophyllin und dem Antikörper erhaltenen Standardkurve bestimmt.

7. Verfahren nach Anspruch 6, dadurch gekennzeichnet, daß das markierte Theophyllin radioaktiv markiertes Theophyllin, vorzugsweise mit $^{125}$Iod iodiertes Theophyllin-7-buttersäure-N-[2-(4-hydroxyphenyl)-ethyl]-amid, ist.

8. Verfahren nach Anspruch 7, dadurch gekennzeichnet, daß das radioaktiv markierte Theophyllin mit $^{125}$Iod iodiertes Theophyllin-7-buttersäure-N-(2-imidazol-4-yl-ethyl)-amid ist.

9. Verfahren nach Anspruch 7, dadurch gekennzeichnet, daß das radioaktiv markierte Theophyllin mit $^{125}$Iod iodiertes Theophyllin-7-butyryl-tyrosin bzw. -tyrosinmethyl- oder -ethylester ist.

10. Verfahren nach Anspruch 6, dadurch gekennzeichnet, daß das markierte Theophyllin enzymmarkiertes Theophyllin, vorzugsweise mit Glucose-6-phosphat-dehydrogenase markiertes Theophyllin ist.

11. Verfahren nach Anspruch 6, dadurch gekennzeichnet, daß das markierte Theophyllin fluoreszenzmarkiertes Theophyllin, vorzugsweise mit 4-Methyl-7-methoxycumarin oder mit Fluorescein markiertes Theophyllin ist.

12. Reagens-Set für die Verwendung in dem Verfahren nach einem der Ansprüche 6 bis 11, dadurch gekennzeichnet, daß es markiertes Theophyllin und Antikörper, gewonnen durch Injektion eines Antigens nach einem der Ansprüche 1 bis 4 in ein Wirtstier, enthält.

13. Verwendung eines Antikörpers, der nach Injektion eines Antigens nach einem der Ansprüche 1 bis 4 in ein Wirtstier erhalten worden ist zur Bestimmung von Theophyllin.

14. Antiserum, erhalten nach Injektion eines Antigens nach einem der Ansprüche 1 bis 4 in ein Wirtstier.

## Claims

1. Antigen, consisting of a compound of general formula I

$$T-(CH_2)_n-COOH \quad (I)$$

wherein T denotes a theophylline-(7) radical and n denotes an integer from 2 to 4, the compound being covalently bonded to an immunogenic carrier via the carboxyl group.

2. Antigen according to Claim 1, characterised in that the immunogenic carrier is a protein.

3. Antigen according to Claim 2, characterised in that the immunogenic carrier is bovine serum albumin.

4. Antigen according to one of Claims 1 to 3, characterised in that in the general formula I n denotes the number 3.

5. Process for the preparation of an antigen according to Claim 1, characterised in that a compound of the general formula I,

$$T-(CH_2)_n-COOH \quad (I),$$

wherein T denotes a theophylline-(7) radical and n denotes an integer from 2 to 4, is esterified with a hydroxy group of an immunogenic carrier or is converted with an amino group of an immunogenic carrier into an acid amide.

6. Process for the determination of theophylline in a sample, characterised in that the sample is mixed with a known amount of labelled theophylline and an antibody which reacts with theophylline and with the labelled theophylline to form a complex and which has been produced by injecting an antigen according to one of Claims 1 to 4 into host animals, the degree of bonding of the labelled theophylline is measured and the amount of theophylline present in the sample is determined from this measurement by comparison with a standard curve obtained by adding known amounts of theophylline to a defined mixture of labelled theophylline and the antibody.

7. Process according to Claim 6, characterised in that the labelled theophylline is a redioactively labelled theophylline, preferably thephylline-(7)-butyric acid N-[2-(4-hydroxyphenyl)-ethyl]-amide iodinated with $^{125}$iodine.

8. Process according to Claim 7, characterised in that the radioactively labelled theophylline is theophylline-(7)-butyric acid N-(2-imidazol-4-yl-

ethyl)-amide iodinated with $^{125}$iodine.

9. Process according to Claim 7, characterised in that the radioactively labelled theophylline is theophylline-(7)-butyryl-tyrosine or -tyrosine methyl ester or ester, iodinated with $^{125}$iodine.

10. Process according to Claim 6, characterised in that the labelled theophylline is enzyme-labelled theophylline, preferably theophylline labelled with glucose-6-phosphate-dehydrogenase.

11. Process according to Claim 6, characterised in that the labelled theophylline is fluorescent-labelled theophylline, preferably theophylline labelled with 7-methoxy-4-methylcoumarin or with fluorescein.

12. Reagent set for use in the process according to Claims 6 to 11, characterised in that it contains labelled theophylline and an antibody obtained by injecting an antigen according to one of Claims 1 to 4 into a host animal.

13. Use of an antibody, obtained by injecting an antigen according to one of Claims 1 to 4 into a host animal, for the determination of theophylline.

14. Antiserum obtained by injecting an antigen according to one of Claims 1 to 4 into a host animal.

**Revendications**

1. Antigène caractérisé en ce qu'il est constitué par un composé qui répond à la formule générale I ci-après:

$$T-(CH_2)_n-COOH \qquad (I)$$

dans laquelle T représente un radical théophylline-7 et n un nombre entier compris entre 2 et 4, le composé étant lié par covalence, par l'intermédiaire du group carboxyle, à un support immunogène.

2. Antigène selon la revendication 1, caractérisé par le fait que le support immunogène est une protéine.

3. Antigène selon la revendication 2, caractérisé par le fait que le support immunogène est l'albumine de sérum de boeuf.

4. Antigène selon l'une quelconque des revendications 1 à 3, caractérisé par le fait que dans la formule générale I, n représente le nombre 3.

5. Procédé de préparation d'un antigène selon la revendication 1, caractérisé par le fait que dans la formule générale I:

$$T-(CH_2)_n-COOH \qquad (I)$$

dans laquelle T représente un radical théophylline-7 et n un nombre entier compris entre 2 et 4, avec un groupe hydroxyle d'un support immunogène ou qu'on le convertit en un amide d'acide avec un groupe amine d'un support immunogène.

6. Méthode de dosage de la théophylline dans un échantillon, caractérisé par le fait que l'on mélange l'échantillon à une quantité connue de théophylline marquée et d'un anticorps qui réagit avec la théophylline pour former un complexe et qui a été produit par injection de l'un des antigènes, selon l'une quelconque des revendications 1 à 4, à des animaux hôtes, que l'on mesure le degré de liaison de la théophylline marquée et qu'à partir de cette mesure, on détermine la quantité de théophylline présente dans l'échantillon, par comparaison avec une courbe étalon que l'on a obtenue en ajoutant des quantités connues de théophylline à un mélange défini de théophylline marquée et de l'anticorps.

7. Méthode selon la revendication 6, caractérisée par le fait que la théophylline marquée est une théophylline marquée par radioactivité, de préférence le N-2-(4-hydroxyphényl)-éthylamide de l'acide théophylline-7-butyrique iodé au moyen d'iode 125.

8. Méthode selon la revendication 7, caractérisée par le fait que la théophylline marquée par radioactivité est le N-(2-imidazol-4-yl-éthyl)-amide d'acide théophylline-7-butyrique iodé au moyen d'iode 125.

9. Méthode selon la revendication 7, caractérisée par le fait que la théophylline marquée par radioactivité est la théophylline-7-butyryltyrosine ou l'ester méthylique ou éthylique de ce composé, iodés au moyen d'iode 125.

10. Méthode selon la revendication 6, caractérisée par le fait que la théophylline marquée est une théophylline marquée par une enzyme, de préférence une théophylline marquée par la glucose-6-phosphate-déshydrogénase.

11. Méthode selon la revendication 6, caractérisée par le fait que la théophylline marquée est une théophylline marquée par fluorescence, de préférence une théophylline marquée par la 4-méthyl-7-méthoxycoumarine ou par la fluorescéine.

12. Trousse de réactifs pour l'utilisation dans la méthode selon l'une quelconque des revendications 6 à 11, caractérisée par le fait qu'elle contient de la théophylline marquée et des anticorps obtenus par injection, à un animal hôte, d'un antigène selon l'une quelconque des revendications 1 à 4.

13. Application d'un anticorps obtenu après injection à un animal hôte d'un anticorps selon l'une quelconque des revendications 1 à 4, au dosage de la théophylline.

14. Antisérum obtenu après injection à un animal hôte d'un antigène selon l'une quelconque des revendications 1 à 4.